## Europäisches Patentamt

## European Patent Office

## Office européen des brevets

(19)

(11) Numéro de publication: **0 063 827**
**B1**

(12) **FASCICULE DE BREVET EUROPEEN**

(45) Date de publication du fascicule du brevet:
**14.08.85**

(21) Numéro de dépôt: **82105394.9**

(22) Date de dépôt: **02.05.80**

(60) Numéro de publication de la demande initiale en application de l'article 76 CBE:

(51) Int. Cl.⁴: **C 07 D  473/06,** C 07 D  473/12, A 61 K  31/52

(54) **1-Isopropyl- et 1-isobutyl-3,7-diméthylxanthine comme médicaments.**

(43) Date de publication de la demande:
**03.11.82 Bulletin 82/44**

(45) Mention de la délivrance du brevet:
**14.08.85 Bulletin 85/33**

(84) Etats contractants désignés:
**CH DE FR IT LI NL**

(56) Documents cités:
**BEILSTEINS HANDBUCH DER ORGANISCHEN CHEMIE, 4ème édition, vol. XXVI, Verlag,Julius Springer 1937, pages 470-471, Berlin DE**

(73) Titulaire: **SOCIETE DES PRODUITS NESTLE S.A., Case postale 353, CH-1800 Vevey (CH)**

(72) Inventeur: **Philippossian, Georges, Avenue de Valmont 18, CH-1010 Lausanne (CH)**
Inventeur: **Enslen, Marc, Rue de Chamblon 25, CH-1400 Yverdon (CH)**

ACTORUM AG

## Description

L'invention concerne le composé 1-isopropyl-3,7-diméthylxanthine.

Elle a trait également à des médicaments contenant la 1-isopropyl- et la 1-isobutyl-3,7-diméthylxanthine.

Les propriétés pharmacologiques connues des xantines sont leur action excitante du système nerveux central, spasmolytique et diurétique.

Des études comportementales chez le rat ont montré de manière tout à fait inattendue que la 1-isopropyl-3,7-diméthylxanthine et la 1-isobutyl-3,7-diméthylxanthine, contrairement aux xanthines psychostimulantes utilisées en thérapeutique telles que par exemple la caféine ou la théophilline, exerçaient un effet sédatif comparable à celui d'un neuroleptique type tels que la chlorpromazine et l'halopéridol.

Les composés ci-dessus peuvent être fabriqués selon la demande de brevet européenne publié No. 19165 dans laquelle leur mode d'obtention et leurs caractéristiques physiques (point de fusion, spectre de résonnance magnétique nucléaire) sont indiqués.

L'invention concerne donc également une composition pharmaceutique contenant une quantité efficace pour l'effet neuroleptique de 1-isopropyl- ou de 1-isobutyl-3,7-diméthylxanthine en combinaison avec support inerte pharmaceutiquement acceptable.

Les médicaments selon l'invention peuvent se présenter sous diverses formes pharmaceutiques contenant les excipients ou véhicules habituels tels que les comprimés, capsules, suppositoires, solutions, suspensions et être administrés par voie orale, sublinguale, rectale, souscutanée, intramusculaire, intraveineuse ou par inhalation à des doses comprises entre 0,02 et 0,2 g par jour.

Ces composés ont une toxicité aiguë chez la souris entre 200 et 300 mg/kg per os (p. o.) et entre 100 et 200 mg/kg par voie intrapéritonéale.

Les exemples suivants illustrent l'invention:

## Exemple 1

La 1-isopropyl- et la 1-isobutyl-3,7-diméthylxanthine ont fait l'objet d'une étude comportementale chez le rat en fonction de l'anxiété induite par un nouvel environnement. Chez le rat, l'anxiété se manifeste par des redressements de celui-ci sur ses deux pattes postérieures, tandis que ses déplacements sont l'expression de l'activité locomotrice.

## Méthode

— L'induction d'un état d'anxiété s'effectue par confrontation de rats mâles «naïfs» Sprague-Dawley (Iffa-Credo, France), pesant 260 à 300 g avec un nouvel environnement constitué par des cages en Macrolon® (30×25 cm) qui se situent dans une chambre insonorisée et climatisée (22° C et 50% d'humidité relative).

— La détermination du nombre de redressements et de déplacements a lieu de façon automatique à partir de cellules photo-électriques à infrarouge qui n'émettent des impulsions électriques que pour les mouvements francs de l'animal et pas pour les mouvements statiques comme ceux de la tête et de la queue et ceci pour des raisons de reproductibilité. Ces cellules photo-électriques quadrillent les cages à deux niveaux différents de façon à distinguer les redressements des déplacements. Le nombre de redressements et de déplacements est compté par les deux séries de cellules photo-électriques, mémorisé et restitué sur une imprimante selon un programme préétabli.

— On administre oralement par intubation oesophagienne le composé testé en solution, l'administration de référence étant constituée par 6 ml/kg d'eau distillé, et ceci 30 minutes avant de placer les animaux dans les cages. Le nombre de redressements et de déplacements est déterminé durant les 15 minutes qui suivent la mise en cage, par rapport à l'effet engendré par l'administration de référence.

## Traitement des résultats

— Le tableau I ci-après montre l'évolution des effets des substances testées sur le nombre de déplacements et de redressements en fonction des doses administrées exprimé en pourcentage des déplacements et redressements comptés pour l'administration de référence.

### Tableau I

| Substance | Dose administrée mg/kg P.O. | Déplacements % de l'administration de référence | Redressements % de l'administration de référence |
|---|---|---|---|
| 1-isobutyl-3,7-diméthylxanthine | 7,5 | 107 | 95 |
| | 10 | 87 | 74 |
| | 12,5 | 82 | 72 |
| | 15 | 64 | 56 |
| | 17,5 | 55 | 46 |
| | 20 | 49 | 35 |
| 1-isopropyl-3,7-diméthylxanthine | 10 | 92 | 89 |
| | 20 | 81 | 80 |
| | 30 | 85 | 64 |
| | 40 | 56 | 36 |
| | 50 | 42 | 24 |
| chlorpromazine | 5 | 109 | 105 |
| | 7,5 | 80 | 90 |
| | 10 | 62 | 70 |
| halopéridol | 0,16 | 104 | 120 |
| | 0,31 | 73 | 73 |
| | 0,63 | 63 | 57 |
| | 0,94 | 52 | 51 |
| | 1,25 | 43 | 40 |
| | 1,56 | 37 | 29 |
| | 2,5 | 6 | 5 |
| chlordiazépoxide | 5 | 85 | 85 |
| | 7,5 | 85 | 72 |
| | 10 | 69 | 57 |
| | 12,5 | 58 | 44 |

– A partir des données précédentes, on établit les droites de régression pour les déplacements et les redressements selon la méthode décrite par Saubrié, P. J. Pharmacol (Paris), 2 (1971), 457–472, l'équation de ces droites de régression correspondant à la moyenne exprimée en % des performances des références en fonction produit par 10 du logarithme des doses (on ne considère que les doses ne diminuant pas l'activité motrice de plus de 75%).

– La comparaison des pentes des droites de régression correspondant aux déplacements et aux redressements est effectuée en portant sur un système d'axes orthonormés en abscisse (d) la valeur de la pente de la droite des déplacements et en ordonnée (r) celle de la pente des redressements. Ces valeurs mettent en évidence la relation effet–dose pour une substance donnée.

– On détermine également le rapport de la pente des déplacements sur celle des redressements qui permet de juger de la spécificité d'effet d'une substance donnée sur l'anxiété.

Le tableau II ci-après montre les résultats obtenus:

Tableau II

| Substance | Déplacements | | Redressements | | Comparaison des pentes $(d_{/r})$ |
|---|---|---|---|---|---|
| | droite de régression | pente ≠ 0 | droite de régression | pente ≠ 0 | |
| 1-isobutyl-3,7-diméthylxanthine | $y = -138,7\,x + 367,5$ | P<0,001 | $y = -135,9\,x + 350,7$ | P<0,001 | N. S. |
| 1-isopropyl-3,7-diméthylxanthine | $y = -48,32\,x + 186,5$ | P<0,05 | $y = -69,75\,x + 225$ | P<0,05 | P<0,05 ($d_{/r}$<1) |
| chlorpromazine | $y = -84,58\,x + 238,6$ | P<0,05 | $y = -67,11\,x + 211,6$ | P<0,05 | N. S. |
| halopéridol | $y = -51,99\,x + 100,9$ | P<0,01 | $y = -59,2\,x + 103,9$ | P<0,01 | N. S. |
| chlordiazépoxide | $y = -66,72\,x + 202,4$ | P<0,01 | $y = -108,3\,x + 273,2$ | P<0,01 | P<0,01 ($d_{/r}$<1) |
| théophylline | $y = 40,54\,x + 61,83$ | P<0,001 | $y = -9,63\,x + 119$ | N. S. | P<0,001 ($d_{/r}$>1) |

Légende:
– La comparaison des pentes est effectuée à l'aide du test «t» aux probabilités P<0,05, P<0,01 et P<0,001.
– N. S. signifie que la pente est non significativement différente de 0 ou que le rapport entre les pentes des déplacements et des redressements est non significativement différent de 1.

Conclusions

La détermination de l'effet d'une substance est donné par sa localisation par rapport à la droite d = r de pente = 1. Les substances situées sur cette droite ont un effet semblable sur les redressements et les déplacements. Les substances pour lesquelles la valeur absolue de la pente des redressements est significativement supérieure à celle des déplacements ont une action spécifique sur l'anxiété.

Le tableau II ci-dessus montre:
– que la 1-isobutyl-3,7-diméthylxanthine a un profil d'action semblable aux neuroleptiques chlorpromazine et halopéridol, c'est un sédatif non spécifique;
– que la 1-isopropyl-3,7-diméthylxanthine a un effet sédatif faiblement anxiolytique du type chlordiazépoxide;
– que la théophylline est psychostimulante et n'a pas d'effet sur l'anxiété.

**Revendications**

1. 1-isopropyl-3,7-diméthylxanthine.
2. Composition pharmaceutique, caractérisée en ce qu'elle contient une quantité efficace pour l'effet neuroleptique de 1-isopropyl-3,7-diméthyl-xanthine en combinaison avec un support inerte pharmaceutiquement acceptable.
3. Composition pharmaceutique, caractérisée en ce qu'elle contient une quantité efficace pour l'effet neuroleptique de 1-isobutyl-3,7-diméthyl-xanthine en combinaison avec un support inerte pharmaceutiquement acceptable.

**Pantentansprüche**

1. 1-Isopropyl-3,7-dimethylxanthin.
2. Pharmazeutische Zusammensetzung, dadurch gekennzeichnet, dass sie eine neuroleptisch wirksame Menge 1-Isopropyl-3,7-dimethylxanthin in Kombination mit einem pharmazeutisch annehmbaren inerten Träger enthält.
3. Pharmazeutische Zusammensetzung, dadurch gekennzeichnet, dass sie eine neuroleptisch wirksame Menge 1-Isobutyl-3,7-dimethylxanthin in Kombination mit einem pharmazeutisch annehmbaren inerten Träger enthält.

**Claims**

1. 1-Isopropyl-3,7-dimethyl xanthine.
2. A pharmaceutical composition containing an effective quantity of 1-isopropyl-3,7-dimethyl xanthine in combination with an inert pharmaceutically acceptable carrier to produce a neuroleptic effect.

3. A pharmaceutical composition containing an effective quantity of 1-isobutyl-3,7-dimethyl xanthine in combination with an inert pharmaceutically acceptable carrier to produce a neuroleptic effect.